(19) Europäisches Patentamt
European Patent Office
Office européen des brevets

(11) Veröffentlichungsnummer: **0 096 795**
**A2**

(12) # EUROPÄISCHE PATENTANMELDUNG

(21) Anmeldenummer: **83105352.5**

(22) Anmeldetag: **31.05.83**

(51) Int. Cl.³: **C 07 D 213/69**

(30) Priorität: **11.06.82 DE 3221948**

(43) Veröffentlichungstag der Anmeldung: **28.12.83**
**Patentblatt 83/52**

(84) Benannte Vertragsstaaten: **CH DE FR GB LI**

(71) Anmelder: **BAYER AG, Zentralbereich Patente, Marken und Lizenzen, D-5090 Leverkusen 1, Bayerwerk (DE)**

(72) Erfinder: **Fürstenwerth, Hauke, Dr., Morgengraben 3, D-5000 Koeln 80 (DE)**
Erfinder: **Mennicke, Winfried, Dr., Steglitzerstrasse 8, D-5090 Leverkusen (DE)**

(54) **N-Arylpyridone, ihre Herstellung und Verwendung.**

(57) Pyridone, die in einer der möglichen tautomeren Formen der allgemeinen Formel

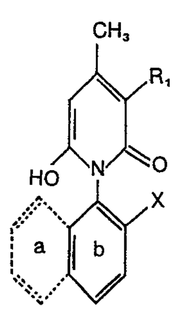

(I)

entsprechen, worin

$R_1$ Wasserstoff oder Acetyl,

X $OR_2$, $SR_3$, $SO_3R_3$ oder einen Rest der Formel

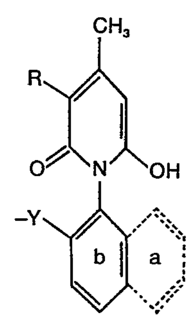

bedeuten,

$R_2$ für Wasserstoff, $C_2$–$C_8$-Alkyl, substituiertes Alkyl, Alkenyl, Cycloalkyl, Aralkyl oder Hetaryl,

$R_3$ für Wasserstoff, Alkyl, Alkenyl, Cycloalkyl, Aralkyl oder Hetaryl und

Y für ein Brückenglied stehen und

worin die Ringe a und b, die cyclischen Reste und der Alkylrest $R_3$ weitersubstituiert sein können, können als Zwischenprodukte zur Herstellung von Farbstoffen – speziell als Kupplungskomponenten – von Pharmaka und Schädlingsbekämpfungsmitteln verwendet werden.

EP 0 096 795 A2

BAYER AKTIENGESELLSCHAFT 5090 Leverkusen, Bayerwerk
Zentralbereich
Patente, Marken und Lizenzen Mi-Klu/c

# N-Arylpyridone, ihre Herstellung und Verwendung

Gegenstand der Erfindung sind Pyridone, die in einer der möglichen tautomeren Formen der allgemeinen Formel

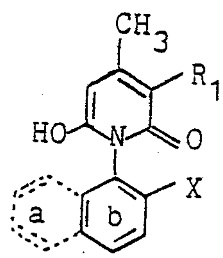

(I)

entsprechen, worin

$R_1$ Wasserstoff oder Acetyl,

X $OR_2$, $SR_3$, $SO_3R_3$ oder einen Rest der Formel

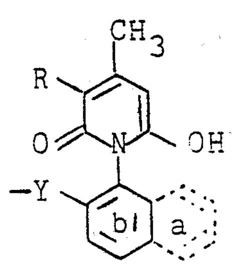

bedeuten,

$R_2$ für Wasserstoff, $C_2$-$C_8$-Alkyl, substituiertes Alkyl, Alkenyl, Cycloalkyl, Aralkyl oder Hetaryl,

$R_3$ für Wasserstoff, Alkyl, Alkenyl, Cycloalkyl, Aralkyl oder Hetaryl und

Y für ein Brückenglied stehen, und

worin die Ringe a und b, die cyclischen Reste und der Alkylrest $R_3$ weitersubstituiert sein können, ihre Herstellung und ihre Verwendung als Zwischenprodukte zur Herstellung von Farbstoffen - speziell als Kupplungskomponenten - von Pharmaka und Schädlingsbekämpfungsmitteln.

In Formel (I) stehen vorzugsweise

Alkyl für einen Rest mit 1 - 12 C-Atomen,
Alkenyl für einen Rest mit 2 - 5 C-Atomen,
Cycloalkyl für Cyclopentyl oder Cyclohexyl,
Aralkyl für Benzyl, Phenylethyl, Phenylpropyl oder Naphthylmethyl und
Hetaryl für Benzthiazolyl, Benzimidazolyl oder Benzoxazolyl.

Als Substituenten dieser Reste kommen vorzugsweise nichtionische Substituenten sowie die Carboxy- und Sulfogruppe infrage.

Hervorzuheben sind Pyridone der Formel (I), worin

X für $X_1$ steht, das die Bedeutung von OH oder SH hat, und

$R_1$ für $R_4$ steht, das die Bedeutung von $COCH_3$ hat.

Von besonderem Interesse sind Pyridone, die in einer der möglichen tautomeren Formen der Formel

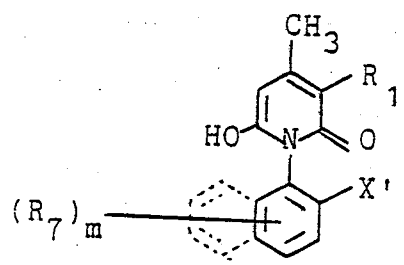

(II)

entsprechen, worin

$R_1$ die in Formel (I) angegebene Bedeutung hat und

X' $OR_2'$, $SR_2'$, $SO_3R_3'$ oder einen Rest der Formel

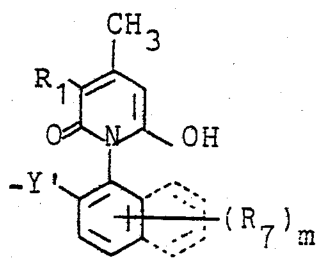

bedeutet,

$R_2'$ und $R_3'$ für Wasserstoff, $C_2$-$C_4$-Alkenyl, gegebenenfalls durch $C_1$-$C_4$-Alkyl substituiertes Cyclohexyl oder Cyclopentyl, gegebenenfalls durch $C_1$-$C_4$-Alkyl, $C_1$-$C_4$-Alkoxy oder Halogen substituiertes Benzyl, Phenylethyl, Benzthiazolyl, Benzimidazolyl oder Benzoxazolyl,

$R_2'$ außerdem für $C_2$-$C_8$-Alkyl oder durch Halogen, Cyan, $C_1$-$C_8$-Alkoxycarbonyl, Carboxy, Cyclohexyl, $OSO_3H$, $SO_3H$, Benzthiazolyl, Benzimidazolyl, Benzoxyzolyl, Benzthiazolylthio, Benzimidazolylthio, Benzoxazolylthio, oder einen Rest der Formel

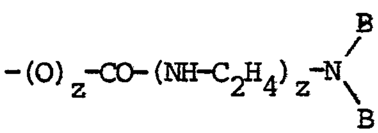 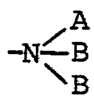 . Anion oder $A-O-(C_2-C_4\text{-Alkylen-O})_n-$

mit

A und B = Wasserstoff und $C_1$-$C_4$-Alkyl,

A = außerdem Phenyl, Benzyl, Phenylethyl, Acetyl, Methylsulfonyl oder Benzoyl oder

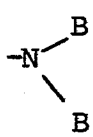 = Piperidin, Morpholin oder Piperazin, die durch $C_1$-$C_4$-Alkyl, Amino-$C_2$-$C_4$-alkyl oder Hydroxy-$C_2$-$C_4$-alkyl substituiert sein können,

m = 1,2 oder 3,

n = 0, 1 oder 2,

z = 0 oder 1,

substituiertes $C_1$-$C_8$-Alkyl,

$R_3'$ außerdem für $C_1$-$C_8$-Alkyl, das durch die bei $R_2'$ genannten Reste substituiert sein kann,

$R_7$ für Wasserstoff, $C_1$-$C_4$-Alkyl, Halogen, Nitro, Cyan, Carboxy, Sulfo, $C_1$-$C_4$-Alkylsulfonyl, Phenylsulfonyl, oder einen Rest der Formel

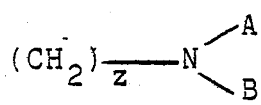 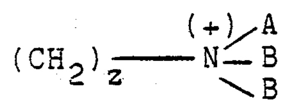 · Anion

oder A-O-($C_2$-$C_4$-Alkylen-O)$_m$- und

Y' für ein Brückenglied der Formel

-D-E-D-

mit

D = O oder S und

E = $C_2$-$C_6$-Alkylen, das durch O, S oder Phenylen unterbrochen sein kann,

stehen.

Unter Halogen wird im Vorstehenden insbesondere Fluor, Chlor oder Brom verstanden.

Besonders bevorzugt sind Pyridone, die in einer der möglichen tautomeren Formen der Formel

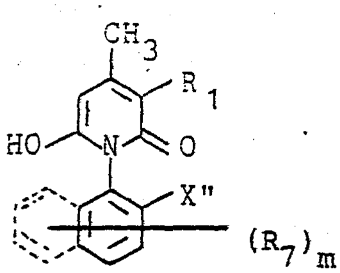

(III)

entsprechen, worin

$R_1$, $R_7$ und m die angegebene Bedeutung haben, und

X" $OR_2''$ oder $SR_3''$ bedeutet,

$R_2''$ und $R_3''$ für H, $-C_2-C_4$-Alkyl, $-CH_2-\underset{C_2H_5}{CH}-C_4H_9$,

$-C_2H_4OH$, $-C_2H_4CN$, $-C_2H_4-O-CH_3$, $-C_2H_4-O-CO-C_1-C_4$-Alkyl, $-C_2H_4-OSO_3H$, $-C_2H_4-SO_3H$, $-CH_2-CO_2H$, $-CH_2-CONH_2$,

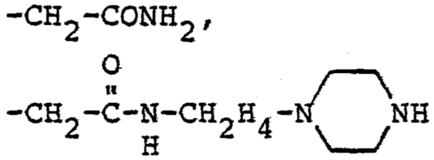, $-(CH_2)_{2\ od.\ 3}N(C_1-C_4\text{-Alkyl})_2$,

$-(CH_2)_{2\ od.\ 3}\overset{(+)}{N}(C_1-C_4\text{-Alkyl})_3$.Anion,

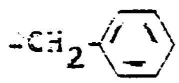 , 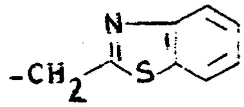 , 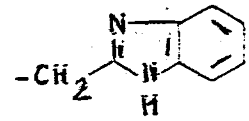

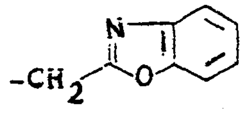 , 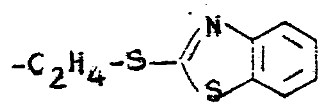 ,

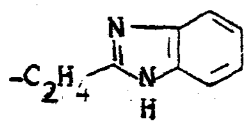 stehen und

$R_3''$ zusätzlich $CH_3$ bedeuten kann.

Zur Herstellung der Verbindungen der Formel I setzt man Verbindungen der Formel

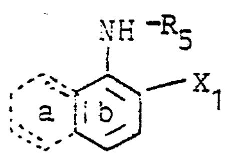 (IV)

worin

$R_5$ für H oder $CO-CH_2-CO-CH_3$ steht und $X_1$ die vorstehend genannte Bedeutung hat,

in wäßrig alkalischem Medium mit Diketen um und erhält

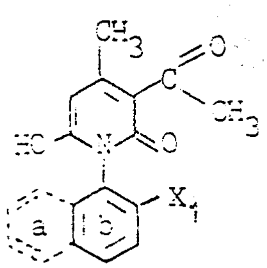 (V)

Durch Umsetzung mit Verbindungen

$R_6$-Y

worin

$R_6$ für Alkyl, Cycloalkyl, Aralkyl oder Hetaryl und

Y für eine als Anion abspaltbare Gruppe stehen,

oder Oxidation lassen sich daraus in üblicher Weise Pyridone der Formel

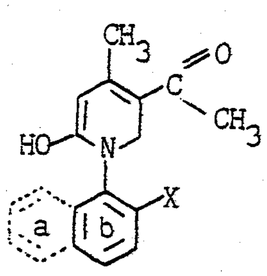

(VI)

herstellen.

Pyridone der Formel VI können durch saure oder alkalische Spaltung in Pyridone der Formel

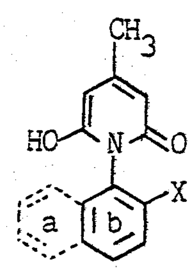

(VII)

überführt werden.

Pyridone der Formel VII werden auch erhalten, wenn man Pyridone der Formel V zunächst entacetyliert und die dabei entstehenden Pyridone der Formel

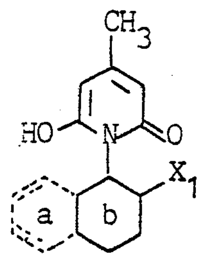

(VIII)

mit Verbindungen der Formel $R_6$-Y umsetzt oder oxidiert.

Zweckmäßigerweise führt man die erfindungsgemäße Reaktion zur Herstellung von Pyridonen der Formel V so durch, daß man eine Verbindung der Formel IV in wäßriger Natronlauge vorlegt und unter kräftigem Rühren Diketen zusetzt. Zweckmäßige Umsetzungstemperaturen liegen im Bereich von -10° bis +30°, vorzugsweise 0-10°. Auf 1 Mol der Verbindungen IV setzt man mindestens 2 Mol, vorzugsweise 2-4 Mol Diketen zu.

Es ist möglich, daß sich bei der Umsetzung von Verbindungen der Formel IV mit Diketen zu Pyridonen der Formel V Arylide der Formel

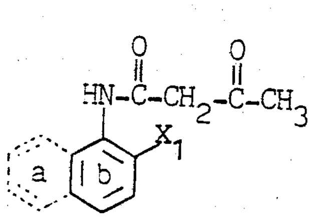

(IX)

intermediär bilden, doch kann nicht ausgeschlossen werden, daß bei dieser Umsetzung Verbindungen der Formel

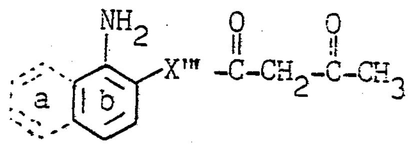

(X)

worin

X"' O oder S bedeuten,

intermediär entstehen und mit weiterem Diketen zu Verbindungen der Formel V reagieren.

Die Umwandlung von Pyridonen der Formel V in Pyridone der Formeln VI und VIII sowie die Überführung von Verbindungen der Formel VI in Verbindungen der Formel VII und von VIII in VII können unter denen für diese im Prinzip bekannten Reaktionen üblichen Bedingungen durchgeführt werden. Auch die Oxidation der SH haltigen Pyridone zu $SO_3H$ haltigen Pyridonen erfolgt unter den üblichen Bedingungen.

Die erfindungsgemäßen Verbindungen der Formel I sind als Zwischenprodukte zur Herstellung von Pharmazeutika und Pflanzenschutzmitteln einzusetzen. Darüber hinaus stellen sie als CH-acide Verbindungen wertvolle Kupplungs- und Kondensationskomponenten dar, insbesondere zur Herstellung von Farbstoffen.

Beispiel 1

115 g (1 Mol) 2-Amino-thiophenol werden in 600 ml 20 %iger Natronlauge gelöst. Unter Kühlung werden 180 ml Diketen langsam so zugetropft, daß die Temperatur des Reaktionsgemisches 5° nicht übersteigt. Man läßt 1 Stunde bei 5° nachrühren und versetzt dann die klare Lösung mit 130 ml Dimethylsulfat, wobei die Temperatur durch Kühlung bei 10-15° gehalten wird. Man rührt 25 Stunden nach, stellt das Reaktionsgemisch auf pH 8, klärt über A-Kohle und tropft die Lösung in eiskalte Salzsäure ein. Der resultierende Niederschlag wird isoliert, mit Wasser neutral gewaschen und getrocknet. Man erhält 230 g Produkt der Formel

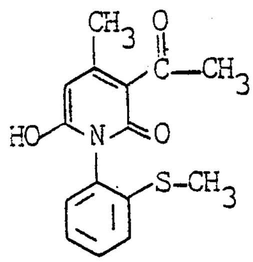

Fp: 183-185° (EtOH)

$^1$H-NMR ($CDCl_3$): $\delta$ = 7.1 - 7.5 (m; 4 H), 6.0 (s; 1H), 2.6 (s; 3 H), 2.45 (s; 6 H)

UV (50 proz. Essigsäure): $\lambda_{max}$ = 332 nm ( $\varepsilon$ = 19200)

UV (verd. Natronlauge): $\lambda_{max}$ = 334 nm ( $\varepsilon$ = 21600)

In den folgenden Beispielen arbeitet man nach Angaben des Beispiels 1, setzt aber statt Dimethylsulfat die in der

Tabelle aufgeführten Alkylierungsmittel R-Y ein.

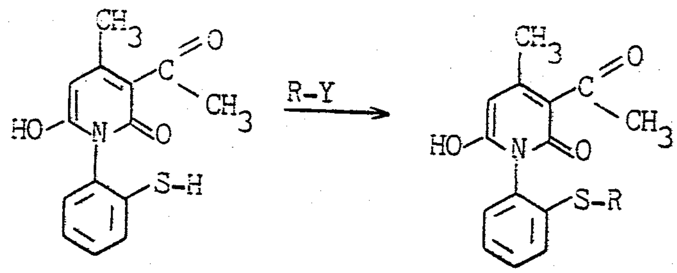

| Bsp. Nr. | R-Y | R |
|---|---|---|
| 2 | $H_5C_2-Br$ | $-C_2H_5$ |
| 3 | 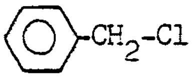 | 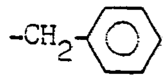 |
| 4 | 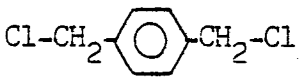 | 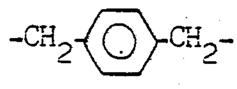 |
| 5 | $Br-C_2H_4-Br$ | $-CH_2-CH_2-$ |
| 6 | $HO-C_2H_4Cl$ | $-CH_2-CH_2-OH$ |
| 7 | $H_9C_4-NH-\underset{\overset{\|}{O}}{C}-O-C_2H_4-Cl$ | $-C_2H_4-O-\underset{\overset{\|}{O}}{C}-\underset{H}{N}-C_4H_9$ |
| 8 | $HO_2C-CH_2-Cl$ | $-CH_2-CO_2H$ |
| 9 | $H_2N-\overset{\overset{O}{\|}}{C}-CH_2-Br$ | $-CH_2-\overset{\overset{O}{\|}}{C}-NH_2$ |
| 10 | $HO_3S-C_2H_4-Cl$ | $-CH_2-CH_2-SO_3H$ |
| 11 | 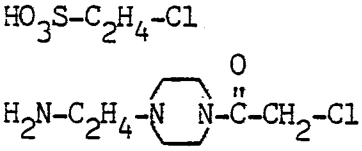 | 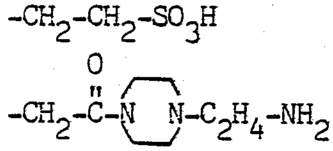 |
| 12 | $(CH_3)_2N-C_2H_4-Cl$ | 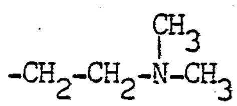 |
| 13 | $(CH_3)_2N-C_3H_6-Cl$ | 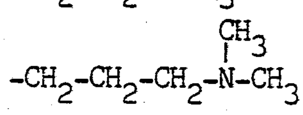 |
| 14 | $(CH_3)_3\overset{(+)}{N}-C_2H_4-Cl\ Cl^{(-)}$ | 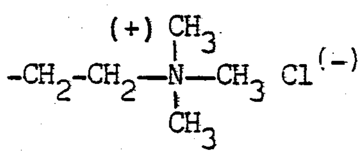 |
| 15 | 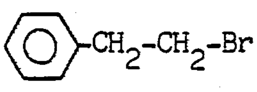 | 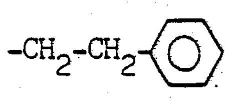 |
| 16 | $CH_2 = CH-CH_2-Cl$ | $-CH_2-CH = CH_2$ |
| 17 | $NC-CH_2-CH_2-Br$ | $-CH_2-CH_2-CN$ |
| 18 | $H_7C_3O-\overset{\overset{O}{\|}}{C}-CH_2-CH_2-Cl$ | $-CH_2-CH_2-\overset{\overset{O}{\|}}{C}-OC_3H_7$ |
| 19 | 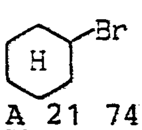 | 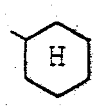 |

20 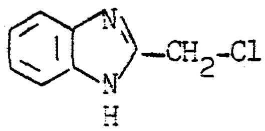 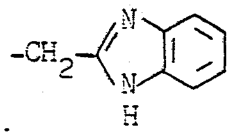

21 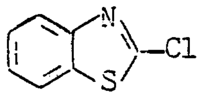 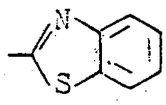

22 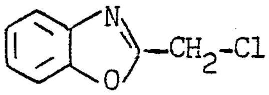 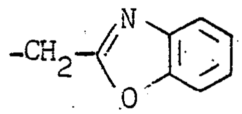

Arbeitet man wie in Beispiel 1 beschrieben, setzt jedoch statt 2-Aminothiophenol die in folgender Tabelle aufgeführten Amino-thiophenole ein und alkyliert mit R-Y so erhält man die aufgeführten Pyridone

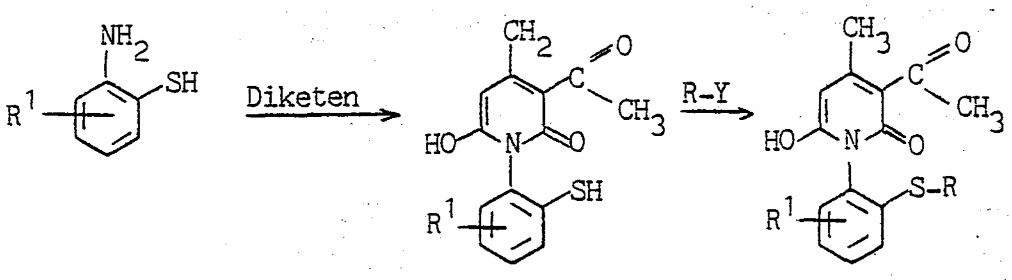


| Beispiel | $R^1$ | R-Y | R |
|---|---|---|---|
| 23 | $5-OCH_3$ | $CH_3-J$ | $-CH_3$ |
| 24 | $5-OC_2H_5$ | $CH_3-OSO_3CH_3$ | $-CH_3$ |
| 25 | $5-CH_3$ | 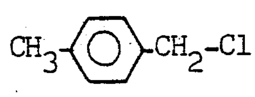 | 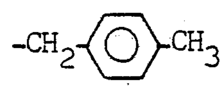 |
| 26 | $4-SO_3H$ | $HO-C_2H_4-Cl$ | $-CH_2-CH_2-OH$ |
| 27 | $5-Cl$ | $H_2N-\underset{\underset{O}{\|}}{C}-CH_2-Br$ | $-CH_2-\overset{\overset{O}{\|}}{C}-NH_2$ |
| 28 | $4-NO_2$ | $H_5C_2-Br$ | $-C_2H_5$ |
| 29 | $5-NO_2$ | 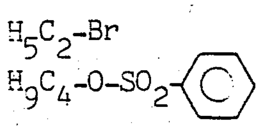 | $-C_4H_9$ |
| 30 | $5-SO_3H$ | $HO_2C-CH_2-CH_2-Br$ | $-CH_2-CH_2-CO_2H$ |

Beispiel 31

1 Mol 2-Amino-thiophenol wird wie in Bsp. 1 beschrieben mit Diketen umgesetzt. Die resultierende Lösung wird auf pH 7 gestellt, mit A-Kohle geklärt und in eiskalte Salzsäure eingerührt. Es resultiert ein öliger Niederschlag, der beim Nachrühren durchkristallisiert. Nach Isolieren und Trocknen erhält man 254 g Produkt der Formel

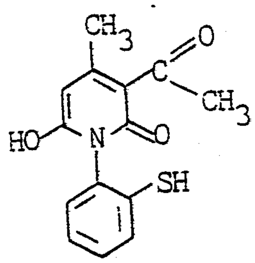

$^1$H-NMR (CDC13): $\delta$ = 7. - 7.7 (m: 4H), 5.9 (s; 1 H) 2.55 (s; 3 H), 2.4 (s; 3 H)

In analoger Weise können die Umsetzungprodukte der substituierten Aminothiophenole der Bsp. 23 - 30 mit Diketen isoliert werden.

Beispiel 32

39 g (0,2 Mol) N-(2-Hydroxyphenyl)-3-oxobutanamid werden in verdünnter Natronlauge gelöst und bei 0 - 10° tropfenweise mit 20 ml Diketen versetzt. Man läßt 1 Stunde bei 5° nachrühren und trägt dann das Reaktionsgemisch auf Salzsäure aus. Nach filtrieren, waschen und trocknen werden 41 g Produkt der Formel

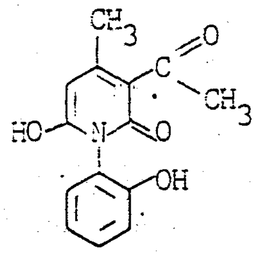

Fp: 225°

erhalten.

UV (50 proz. Essigsäure): $\lambda_{max}$ = 325 nm, $\varepsilon$ = 19050
UV (verd. Natronlauge): $\lambda_{max}$ = 344 nm, $\varepsilon$ = 21500

Beispiel 33

22 g (0,2 Mol) 2-Aminophenol werden in verdünnter Natronlauge gelöst und bei 0-5° tropfenweise mit 50 ml Diketen versetzt. Man läßt 1 Stunde bei 10° nachrühren und trägt dann das Reaktionsgemisch auf Salzsäure aus. Nach Aufarbeiten werden 40 g Produkt erhalten, welches mit den in Bsp. 32 beschrieben identisch ist.

In den folgenden Beispielen arbeitet man nach den Angaben des Bsp. 33, setzt aber statt 2-Aminophenol die in der Tabelle aufgeführten Aminophenole ein und erhält die angegebenen 3-Acetyl-6-hydroxy-2-pyridone.

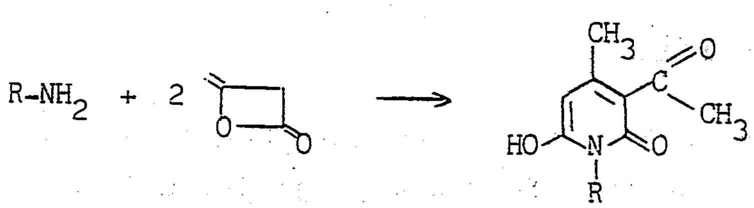

| Beispiel | R | Beispiel | R |
|---|---|---|---|
| 34 | 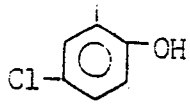 | 38 | 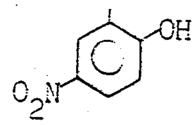 |
| 35 | 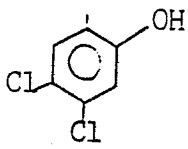 | 39 | 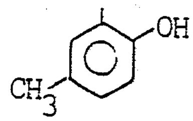 |
| 36 | 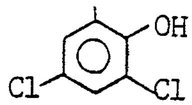 | 40 | 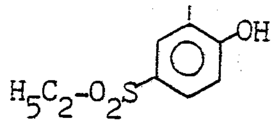 |
| 37 | 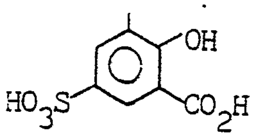 | 41 | 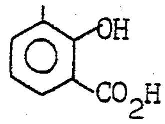 |
| 42 | 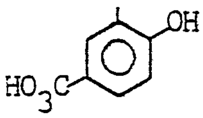 | 49 | 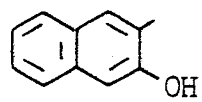 |
| 43 | 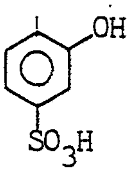 | 50 | 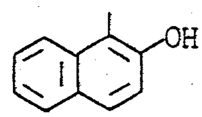 |
| 44 | 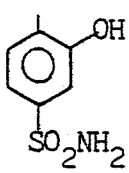 | 51 | 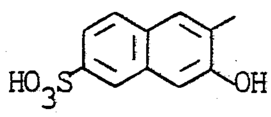 |
| 45 | 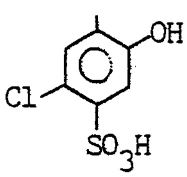 | 52 | 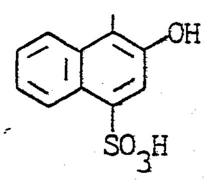 |
| 46 | 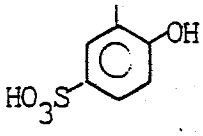 | 53 | 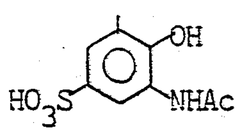 |
| | | 54 | 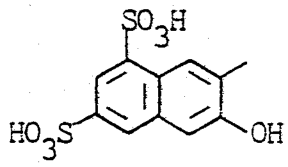 |

| Beispiel | R | Beispiel | R |
|---|---|---|---|
| 47 | 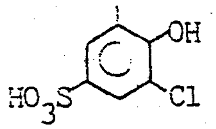 | 55 | 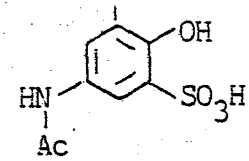<br>Ac = Acetyl |
| 48 | 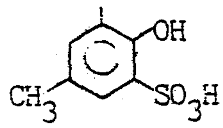 | | |

<u>Beispiel 56</u>

193 g (1 Mol) N-(2-Hydroxyphenyl)-3-oxobutanamid werden wie in Bsp. 32 beschrieben mit Diketen umgesetzt. Nach einstündigem Nachrühren wird das alkalische Reaktionsgemisch mit 200 ml Dimethylsulfat versetzt. Man rührt 20 Stunden bei 10-15° nach. Dann wird mit A-Kohle bei pH 9 geklärt und das Produkt durch Austragen der Lösung auf Salzsäure gefällt. Nach Aufarbeitung erhält man reines Produkt der Formel

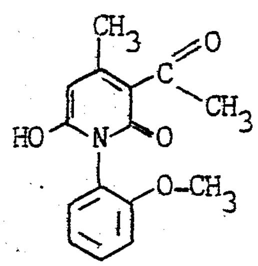

Fp: 198°

$^1$H-NMR ($CDCl_3$): $\delta$ = 7.4 - 6.8 (m; 4H), 5.95 (s; 1 H) 4,75 (s; 3 H), 2.55 (s; 3 H), 2.3 (s; 3 H)

UV (50 proz. Essigsäure): $\lambda_{max}$ = 325 nm ($\varepsilon$ = 17500)

UV (verd. Natronlauge): $\lambda_{max}$ = 342 nm ($\varepsilon$ = 19500)

Zum identischen Produkt gelant man, wenn man das gemäß Bsp. 33 hergestellte und isolierte Pyridon in alkalischer Lösung mit Dimethylsulfat umsetzt. Analog lassen sich die in den Beispielen 34 - 55 beschriebenen Pyridone methylieren oder analog der Beispiele 2 - 30 mit den Verbindungen R-Y umsetzen.

Beispiel 57

150 g der Verbindungen

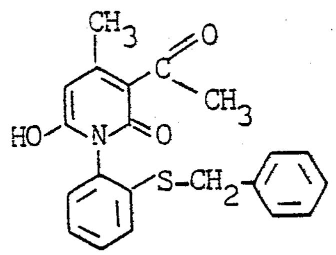

werden in 1 l 20%iger Schwefelsäure 4 Stunden am Rückfluß gekocht. Man kühlt auf 25° ab und isoliert das ausgeschiedene Produkt. Nach Umkristallisieren aus i-Propanol erhält man 85 g reines Produkt der Formel

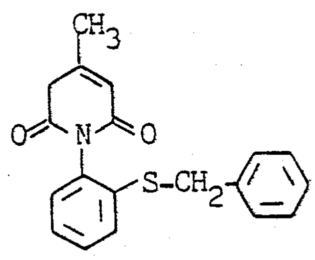

Fp: 134 - 135°

$^1$H-NMR ($CDCl_3$): $\delta$ = 7.1 - 7.4 (m; 9 H), 6.1 (d; 1 H)
4.0 (s; 2 H), 3.4 (s; 2 H), 2,0 (s; 3H)

Die in den Beispielen 1 - 56 aufgeführten Pyridone lassen sich in analoger Weise entacetylieren.

Patentansprüche

1. Pyridone, die in einer der möglichen tautomeren Formen der allgemeinen Formel

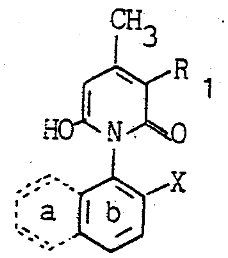

entsprechen, worin

$R_1$ Wasserstoff oder Acetyl,

X $OR_2$, $SR_3$, $SO_3R_3$ oder einen Rest der Formel

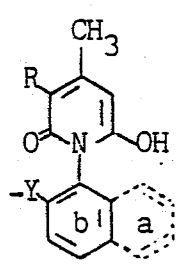

bedeuten,

$R_2$ für Wasserstoff, $C_2$-$C_8$-Alkyl, substituiertes Alkyl, Alkenyl, Cycloalkyl, Aralkyl oder Hetaryl,

$R_3$ für Wasserstoff, Alkyl, Alkenyl, Cycloalkyl, Aralkyl oder Hetaryl und

Y für ein Brückenglied stehen, und

worin die Ringe a und b, die cyclischen Reste und der Alkylreste $R_3$ weitersubstituiert sein können.

2. Pyridone der Formel des Anspruchs 1, worin

X für $X_1$ steht, das die Bedeutung von OH oder SH hat, und

$R_1$ für $R_4$ steht, das die Bedeutung von $COCH_3$ hat.

3. Pyridone, die in einer der möglichen tautomeren Formen der Formel

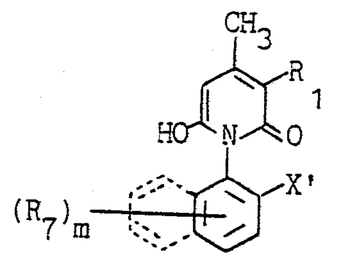

entsprechen, worin

$R_1$ die in Anspruch 1 angegebene Bedeutung hat, und

X' $OR'_2$, $SR'_3$, $SO_3R'_3$ oder einen Rest der Formel

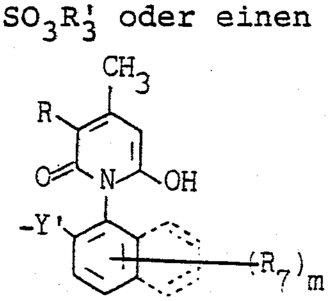

bedeutet,

$R_2'$ und $R_3'$ für Wasserstoff, $C_2$-$C_4$-Alkenyl, ggf. durch $C_1$-$C_4$-Alkyl substituiertes Cyclohexyl oder Cyclopentyl, ggf. durch $C_1$-$C_4$-Alkyl, $C_1$-$C_4$-Alkoxy oder Halogen substituiertes Benzyl, Phenylethyl, Benzthiazolyl, Benzimidazolyl oder Benzoxazolyl,

$R_2'$ außerdem für $C_2$-$C_8$-Alkyl oder durch Halogen, Cyan, $C_1$-$C_8$-Alkoxycarbonyl, Carboxy, $SO_3H$, $OSO_3H$, Cyclohexyl, Benzimidazolyl, Benzthiazolyl, Benzoxazolyl, Benzthiazolylthio, Benzimidazolthio, Benzoxazolylthio, oder einen Rest der Formel

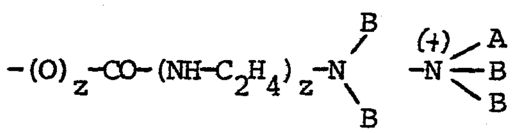 . Anion oder A-O-($C_2$-$C_4$-Alkylen-O)$_n$-

mit

A und B = Wasserstoff und $C_1$-$C_4$-Alkyl,

A = außerdem Phenyl, Benzyl, Phenylethyl, Acetyl, Methylsulfonyl oder Benzoyl, oder

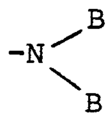 = Piperidin, Morpholin oder Piperazin, die durch $C_1$-$C_4$-Alkyl, Amino-$C_2$-$C_4$-alkyl oder Hydroxy-$C_2$-$C_4$-alkyl substituiert sein können,

m = 1, 2 oder 3,

n = 0, 1 oder 2,

z = 0 oder 1,
substituiertes $C_1$-$C_8$-Alkyl,

$R_3'$ außerdem für $C_1$-$C_8$-Alkyl, das durch die bei $R_2'$ genannten Reste substituiert sein kann,

$R_7$ für Wasserstoff, $C_1$-$C_4$-Alkyl, Halogen, Nitro, Cyan, Carboxy, Sulfo, $C_1$-$C_4$-Alkylsulfonyl, Phenylsulfonyl oder einen Rest der Formel

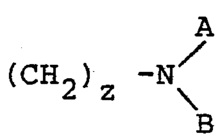 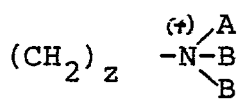 . Anion

oder A-O-($C_2$-$C_4$-Alkylen-O)$_m$- und

Y' für ein Brückenglied der Formel

-D-E-D-

mit

D = 0 oder S und

E = $C_2$-$C_6$-Alkylen, das durch O, S oder Phenylen unterbrochen sein kann,

stehen.

4. Pyridone die in einer der möglichen tautomeren Formen der Formel

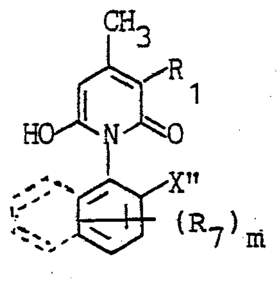

entsprechen, worin

$R_1$, $R^7$ und m die vorstehend angegebene Bedeutung haben und

X" $OR_2''$ oder $SR_3''$ bedeutet,

$R_2''$ und $R_3''$ für H, $C_2$-$C_4$-Alkyl, $-CH_2-CH-C_4H_9$, $-C_2H_4OH$,

$C_2H_5$ (an CH)

$C_2H_4CN$, $-C_2H_4-O-CH_3$, $-C_2H_4-O-CO-C_1-C_4$-Alkyl,

$-C_2H_4-OSO_3H$, $-C_2H_4-SO_3H$, $-C_2H_4-O-SO_3H$, $-CH_2-CO_2H$,

$-CH_2-CONH_2$,

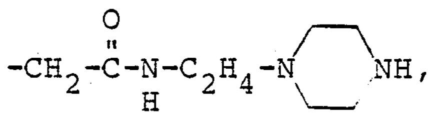

$-(CH_2)_{\overline{2\ od.\ 3}}N(C_1-C_4\text{-Alkyl})_2$

$-(CH_2)_{\overline{2\ od.\ 3}}\overset{(+)}{N}(C_1-C_4\text{-Alkyl})_3.\text{Anion}$

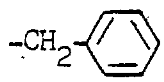 , 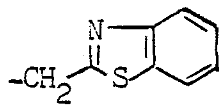 , 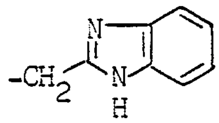 ,

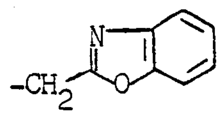 , 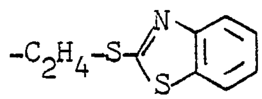 , 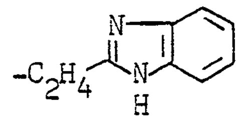

stehen und

$R_3''$ zusätzlich $-CH_3$ bedeuten kann.

5. Verfahren zur Herstellung von Pyridonen des Anspruchs 2, dadurch gekennzeichnet, daß man Amine der allgemeinen Formel

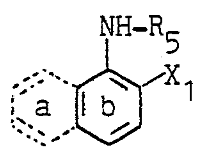

worin

$R_5$ für H oder $-CO-CH_2-CO-CH_3$ steht, und

$X_1$ die in Anspruch 2 angegebene Bedeutung hat,

in wäßrig-alkalischem Medium mit Diketen umsetzt.

6. Verfahren zur Herstellung von Pyridonen der Formel des Anspruchs 1, worin X für $X_2$ steht, das die Bedeutung von $OR_6$ oder $SR_6$ hat, worin

$R_6$ für Alkyl, Cycloalkyl, Aralkyl oder Hetaryl steht,

dadurch gekennzeichnet, daß man die Pyridone des Anspruchs 2 mit Verbindungen der allgemeinen Formel

$$R_6Y$$

umsetzt, worin Y eine als Anion abspaltbare Gruppe ist.

7. Verwendung der Pyridone gemäß den Ansprüchen 1 und 2 und der nach Anspruch 6 hergestellten Pyridone zur Herstellung von Farbstoffen, Pharmaka und Pflanzenschutzmitteln.